Europäisches Patentamt

European Patent Office

Office européen des brevets

(19)

(11) **EP 0 699 661 A2**

(12) **EUROPÄISCHE PATENTANMELDUNG**

(43) Veröffentlichungstag:
06.03.1996 Patentblatt 1996/10

(51) Int. Cl.[6]: **C07C 309/10**, C07C 303/22,
C07C 303/44

(21) Anmeldenummer: 95113205.9

(22) Anmeldetag: 23.08.1995

(84) Benannte Vertragsstaaten:
**AT BE CH DE ES FR GB IT LI NL SE**

(30) Priorität: **01.09.1994 DE 4431056**

(71) Anmelder: **HOECHST AKTIENGESELLSCHAFT
D-65926 Frankfurt am Main (DE)**

(72) Erfinder:
• **Delpy, Klaus, Dr.
D-63128 Dietzenbach (DE)**
• **Engelhardt, Fritz, Dr.
D-60386 Frankfurt am Main (DE)**
• **Zerrer, Ralf, Dr.
D-63755 Alzenau (DE)**
• **Bühring, Dirk, Dr.
D-60529 Frankfurt am Main (DE)**

(54) **Verfahren zur Herstellung von Ethersulfonaten**

(57) Die vorliegende Erfindung betrifft ein Verfahren zur Herstellung von hochreinen weitgehend Fremdsalz-freien Ethersulfonaten der allgemeinen Formel I

$$R^1\text{-}[O\text{-}(CR^2R^{2'}\text{-}CR^3R^{3'})_x]_y\text{-}OCR^4R^{4'}CR^5R^{5'}SO_3M \quad I$$

worin
$R^2$ bis $R^5$, $R^{2'}$ bis $R^{5'}$, M, x und y wie in Anspruch 1 angegeben definiert sind
durch Umsetzung einer Verbindung der allgemeinen Formel II

$$R^1\text{-}[O\text{-}(CR^2R^{2'}CR^3R^{3'})_x]_y\text{-}OH \quad II$$

mit einer Verbindung der allgemeinen Formel III

$$HO\text{-}CR^4R^{4'}CR^5R^{5'}\text{-}SO_3M \quad III$$

in Gegenwart einer Verbindung der allgemeinen Formel IV

$$MOH \quad IV$$

und anschließender Neutralisation der Verbindung der allgemeinen Formel IV mit einer Säure, dadurch gekennzeichnet, daß als Säure eine Verbindung der allgemeinen Formel V

$$R^1\text{-}[O\text{-}(CR^2R^{2'}\text{-}CR^3R^{3'})_x]_y\text{-}OCR^4R^{4'}CR^5R^{5'}SO_3H \quad V$$

eingesetzt wird.

EP 0 699 661 A2

## Beschreibung

Die vorliegende Erfindung betrifft ein Verfahren zur Herstellung von hochreinen, weitgehend Fremdsalz-freien Ethersulfonaten durch Umsetzung von Alkoholen mit Hydroxyalkylsulfonsäuresalzen unter basischer Katalyse.

Ethersulfonate sind wichtige chemische Zwischenprodukte und werden für eine Reihe industrieller Umsetzungen gebraucht.

Demgemäß sind bereits Verfahren zu ihrer Herstellung bekannt. So beschrieben beispielsweise die US 2,535,677, US 4,091,014, US 4,226,807 und US 4,465,602 die Umsetzung von Alkoholen mit Hydroxyalkylsulfonsäuresalzen unter basischer Katalyse, wobei nach Reaktionsende der basische Katalysator durch Säurezusatz neutralisiert werden muß. Das dabei entstehende Salz verbleibt in dem Endprodukt, so daß hochreine, insbesondere Fremdsalz-freie Produkte nach diesen Verfahren nicht erhalten werden können. Ein Verfahren, das hochreine, weitgehend Fremdsalz-freie Ethersulfonate liefert, ist bis heute nicht bekannt, obwohl damit erhebliche Vorteile verbunden wären.

Es wurde nunmehr überraschend gefunden, daß hochreine, weitgehend Fremdsalz-freie Ethersulfonate durch Umsetzung von Alkoholen mit Hydroxyalkansulfonsäuresalzen unter basischer Katalyse erhalten werden können, wenn nach Abschluß der Reaktion mit der zum hergestellten Ethersulfonat konjugierten Ethersulfonsäure neutralisiert wird.

Die vorliegende Erfindung betrifft somit ein Verfahren zur Herstellung von hochreinen, weitgehend Fremdsalzfreien Ethersulfonaten der allgemeinen Formel I

$$R^1\text{-}[O\text{-}(CR^2R^{2'}\text{-}CR^3R^{3'})_x]_y\text{-}OCR^4R^{4'}CR^5R^{5'}SO_3M \quad I$$

worin

| | |
|---|---|
| $R^1$ | Wasserstoff, $(C_1\text{-}C_{22})$-Alkyl, $(C_2\text{-}C_{22})$-Alkenyl, $(C_3\text{-}C_8)$-Cycloalkyl, $(C_6\text{-}C_{14})$-Aryl oder $(C_6\text{-}C_{14})$-Aryl-$(C_1\text{-}C_8)$-Alkyl bedeutet; |
| $R^2$ bis $R^5$ | sowie $R^{2'}$ bis $R^{5'}$ unabhängig voneinander Wasserstoff oder $(C_1\text{-}C_4)$-Alkyl bedeuten; |
| M | ein Alkalimetall, die Ammoniumgruppe oder eine substituierte Ammoniumgruppe bedeutet; |
| x | eine ganze Zahl von 1 bis 20 bedeutet; und |
| y | eine ganze Zahl von 0 bis 20 bedeutet, |

wobei für den Fall x>1 auch die Reste $R^2$, die Reste $R^{2'}$, die Reste $R^3$ und die Reste $R^{3'}$ jeweils unabhängig voneinander sind und wobei $R^1$ nicht Wasserstoff sein kann, wenn y = O durch Umsetzung einer Verbindung der allgemeinen Formel II

$$R^1\text{-}[O\text{-}(CR^2R^{2'}CR^3R^{3'})_x]_y\text{-}OH \quad II$$

mit einer Verbindung der allgemeinen Formel III

$$HO\text{-}CR^4R^{4'}CR^5R^{5'}\text{-}SO_3M \quad III$$

in Gegenwart einer Verbindung der allgemeinen Formel IV

$$MOH \quad IV$$

und anschließender Neutralisation der Verbindung der allgemeinen Formel IV mit einer Säure, dadurch gekennzeichnet, daß als Säure eine Verbindung der allgemeinen Formel V

$$R^1\text{-}[O\text{-}(CR^2R^{2'}\text{-}CR^3R^{3'})_x]_y\text{-}OCR^4R^{4'}CR^5R^{5'}SO_3H \quad V$$

eingesetzt wird.

Alkylgruppen können geradkettig oder verzweigt sein und sind beispielsweise Methyl, Ethyl, n-Propyl, i-Propyl, n-Butyl, i-Butyl, sek.Butyl, tert.Butyl, n-Pentyl, 3-Methylbutyl, Pentyl-3, n-Hexyl, 2-Ethylbutyl oder 2-Ethylhexyl.

Für $R^1$ stehendes Alkyl hat bevorzugt 1 bis 5, besonders bevorzugt 1 bis 3 Kohlenstoffatome. Für $R^2$ bis $R^5$ bzw. $R^{2'}$ bis $R^{5'}$ stehendes Alkyl hat bevorzugt 1 bis 3 Kohlenstoffatome.

Cycloalkyl ist insbesondere Cyclopropyl, Cyclobutyl, Cyclopentyl, Cyclohexyl, Cycloheptyl und Cyclooctyl, wobei Cyclopentyl und Cyclohexyl bevorzugt sind. Unter Cycloalkyl wird aber auch beispielsweise Dimethylcycloalkyl verstanden.

Auch Alkenylgruppen können geradkettig oder verzweigt sein und entsprechen beispielsweise den oben genannten Alkylgruppen. Bevorzugte Alkenylgruppen haben 2 bis 5 Kohlenstoffatome, wobei Vinyl und Allyl besonders bevorzugt sind.

Arylgruppen sind bevorzugt Phenyl, Napthyl, Biphenyl oder Fluorenyl, wobei Phenyl besonders bevorzugt ist.

Bevorzugte Arylalkylgruppen sind Benzyl und Phenethyl.

Ein für M stehendes Alkalimetall ist bevorzugt Natrium und Kalium.

Als für M stehende substituierte Ammoniumgruppe können alle üblichen mono-, di-, tri-, oder tetrasubstituierten Ammoniumgruppen eingesetzt werden.

Eine für M stehende Ammoniumgruppe hat bevorzugt die Formel $^{\oplus}NR^6R^7R^8R^9$, wobei $R^6$, $R^7$, $R^8$ und $R^9$ unabhängig voneinander für Wasserstoff, $(C_1\text{-}C_6)$-Alkyl, Phenyl, substituiertes Phenyl oder Halogen bedeuten. Besonders bevorzugt ist Tetra-$(C_1\text{-}C_4)$-alkylammonium.

In bevorzugt gemäß dem erfindungsgemäßen Verfahren herzustellenden Verbindungen der allgemeinen Formel I bedeuten

| | |
|---|---|
| $R^1$ | Wasserstoff oder $(C_1\text{-}C_6)$-Alkyl |
| $R^2$ bis $R^5$ | bzw. $R^{2'}$ bis $R^{5'}$ unabhängig voneinander Wasserstoff oder $(C_1\text{-}C_3)$-Alkyl |
| M | Natrium oder Kalium |

x        eine ganze Zahl von 1 bis 4 und

y        eine ganze Zahl von 1 bis 10

In besonders bevorzugt gemäß dem erfindungsgemäßen Verfahren herzustellenden Verbindungen der allgemeinen Formel I bedeuten

| | |
|---|---|
| $R^1$ | Wasserstoff |
| $R^2$, $R^{2'}$, $R^3$ und $R^{3'}$ | unabhängig voneinander Wasserstoff oder Methyl, |
| $R^4$, $R^{4'}$, $R^5$ und $R^{5'}$ | Wasserstoff, |
| M | Natrium oder Kalium und |
| x und y | die Zahl 1. |

Das erfindungsgemäße Verfahren ist eine basekatalysierte Kondensationsreaktion, das insbesondere bei Temperaturen von 150 bis 250°C, besonders bevorzugt 180°C unter Normaldruck durchgeführt wird. Es ist bevorzugt, dem Reaktionsgemisch das während der Reaktion entstehende Wasser laufend destillativ zu entziehen. Im allgemeinen betragen die Reaktionszeiten 2 bis 10 Stunden.

In einer bevorzugten Ausführungsform des erfinderischen Herstellungsverfahrens werden die Verbindung der allgemeinen Formel II in einer Menge von 200 bis 700 Mol%, die Verbindung der allgemeinen Formel III in einer Menge von 100 Mol% und die Verbindung der allgemeinen Formel IV in einer Menge von 10 bis 20 Mol% eingesetzt.

In einer weiteren bevorzugten Ausführungsform wird nach Neutralisation des Reaktionsgemisches mit einer Verbindung der allgemeinen Formel V die im Überschuß eingesetzte Verbindung der allgemeinen Formel III mittels eines Dünnschichtverdampfers abgetrennt. Bevorzugt werden dabei ein Druck von 10 bis 500 mbar, sowie eine Temperatur von 120 bis 250°C eingehalten.

Die Verbindungen der allgemeinen Formeln II und III sind bekannt, können käuflich erworben werden und/oder sind nach dem Fachmann bekannten Herstellungsverfahren zugänglich.

Die Verbindungen der allgemeinen Formel V können in einfacher Weise dadurch erhalten werden, daß eine Verbindung der allgemeinen Formel I aus einem vorhergehenden Reaktionsansatz in dem Fachmann bekannter Weise mit einer Säure geeigneten $pK_s$-Werts versetzt und das Produkt isoliert wird. In der Regel sind Säuren wie Schwefelsäure, Salzsäure oder Methansulfonsäure geeignet.

Liegt noch keine Verbindung der allgemeinen Formel I vor, so können auch auf anderen Wegen hergestellte Verbindungen der allgemeinen Formel V eingesetzt werden.

Die nach dem erfindungsgemäßen Verfahren hergestellten Verbindungen der allgemeinen Formel I sind hochrein und weitgehend Fremdsalz-frei. Weitgehend Fremdsalz-frei heißt im Rahmen dieser Erfindung, daß der Fremdsalzgehalt der erfindungsgemäß hergestellten Verbindungen der allgemeinen Formel I höchstens 2 Gew.-% beträgt.

Beispiel 1

Reaktion und Neutralisation

In einem 1-L-Dreihalskolben mit KPG-Rührer, einer beheizbaren 40-cm-Kolonne mit aufgesetztem Liebig-Kühler und Innenthermometer werden
310,1 g (5,0 Mol) Ethandiol-1,2,
148,1 g (1,0 Mol) 2-Hydroxyethansulfonsäure-Na-Salz (Na-isethionat) sowie
4,0 g (0,1 Mol) Natriumhydroxid vorgelegt.

Das Reaktionsgemisch wird unter ständigem Rühren auf eine Innentemperatur von 190 - 195°C erhitzt. Die Manteltemperatur der Destillationskolonne wird bei 110 - 120°C gehalten. Innerhalb von 3 h destillieren ca. 18,0 g Wasser aus dem Reaktionsgemisch ab. Nach dem Abkühlen auf RT wird die alkalische Produktlösung durch Versetzen mit ca. 17,0 g (0,1 Mol)2-(2-hydroxyethoxy)-ethansulfonsäure unter ständigem Rühren neutralisiert.

Aufarbeitung

Aus der glykolhaltigen Produktlösung wird das Ethersulfonat mittels einer Dünnschichtverdampfung isoliert. Hierbei wird die Mantelheizung des Verdampferrohres bei ca. 200°C gehalten. Das Vakuum wurde auf 10 mbar geregelt. Das 2-(2-hydroxyethoxy)ethansulfonsäure-Na-Salz (Diglykolsulfonsäure-Na-Salz) fällt als farbloser Destillationsrückstand mit einem Schmelzbereich von 140 -150°C an. Der Glykolgehalt liegt unter 1 Gew.-%. Der Reingehalt beträgt 98,2 Gew.-%. Der Fremdsalzgehalt beträgt 0,3 Gew.-%.

Wird wie vorstehend gearbeitet, die erhaltene alkalische Produktlösung aber wie gemäß Stand der Technik üblich mittels 9,6 g (0,1 Mol) Methansulfonsäure neutralisiert, liegt der Reingehalt an Ethersulfonat bei 92 bis 94 Gew.-% und der Fremdsalzgehalt bei 6 bis 8 Gew.-%.

Beispiel 2

Analog Beispiel 1 werden
229,0 g (3,0 Mol) Propandiol-1,2,
148,1 g (1,0 Mol) 2-Hydroxyethansulfonsäure-Na-Salz sowie
4,0 g (0,1 Mol) Natriumhydroxid umgesetzt.

Nach Abschluß der Reaktion erfolgt die Neutralisation mit der zum Produkt konjugierten Sulfonsäure und eine entsprechende Aufarbeitung mittels Dünnschichtverdampfer. Der Reinheitsgrad beträgt 98,5 Gew%, der Fremdsalzgehalt 0,8 Gew.-%.
Wird wie vorstehend gearbeitet, die erhaltene alkalische Produktlösung aber wie gemäß Stand der Technik üblich mittels Methansulfonsäure neutralisiert, beträgt der Reingehalt an Ethersulfonat 93,5 Gew.-%, der Fremdsalzgehalt 5,4 Gew.-%.

## Beispiel 3

Analog Beispiel 1 werden
390,3 g (3,0 Mol) 2-Ethylhexanol,
148,1 g (1,0 Mol) 2-Hydroxyethansulfonsäure-Na-Salz sowie
8,0 g (0,2 Mol) Natriumhydroxid umgesetzt.

Die Reaktionstemperatur beträgt 210-220°C, die Reaktionszeit 6 h. Die Manteltemperatur der beheizten Kolonne wird auf 160 - 180°C geregelt. Nach Abschluß der Reaktion erfolgt die Neutralisation mit der zum Produkt konjugierten Sulfonsäure, sowie eine Aufarbeitung mittels Dünnschichtverdampfer wie unter Beispiel 1 beschrieben.

Der Reinheitsgrad beträgt 98 Gew.-%, der Fremdsalzgehalt 1,3 Gew.-%.

Wird wie vorstehend gearbeitet, die erhaltene alkalische Produktlösung aber wie gemäß Stand der Technik üblich mittels Methansulfonsäure neutralisiert, beträgt der Reingehalt an Ethersulfonat 90,6 Gew.-%, der Fremdsalzgehalt bei 8,5 Gew.-%.

## Beispiel 4

Analog Beispiel 1 werden
296,2 g (4,0 Mol) n-Butanol
148,1 g (1,0 Mol) 2-Hydroxyethansulfonsäure-Na-Salz sowie
4,0 g (0,2 Mol) Natriumhydroxid umgesetzt.

Die Reaktionstemperatur beträgt 205-210°C, die Reaktionszeit 8 h.
Die Manteltemperatur der Kolonne wird auf 105-110°C geregelt.

Nach Neutralisation mit der zum Produkt konjugierten Sulfonsäure und Aufarbeitung mittels Dünnschichtverdampfer fällt das Produkt in einer Reinheit von 98,5 Gew.-% an, der Fremdsalzgehalt beträgt 0,6 Gew.-%.

Neutralisation mit Methansulfonsäure gemäß Stand der Technik ergibt einen Reingehalt von 88,5 Gew.-% und einen Fremdsalzgehalt von 10,5 Gew.-%.

## Beispiel 5

Analog Beispiel 1 werden
530,2 g (5,0 Mol) n-Butanol
148,1 g (1,0 Mol) 2-Hydroxyethansulfonsäure-Na-Salz sowie
8,0 g (0,2 Mol) Natriumhydroxid umgesetzt.

Die Reaktionstemperatur beträgt 200-210°C, die Reaktionszeit 6 h.
Die Manteltemperatur der Kolonne wird auf 160-180°C geregelt.

Nach Neutralisation mit der zum Produkt konjugierten Sulfonsäure und anschließender Aufarbeitung mittels Dünnschichtverdampfer fällt das Produkt in einer Reinheit von 98,1 Gew.-% an, der Fremdsalzgehalt beträgt 0,2 Gew.-%.

Neutralisation mit Methansulfonsäure gemäß Stand der Technik ergibt einen Reingehalt von 90 Gew.-% und einen Fremdsalzgehalt von 8 Gew.%.

## Patentansprüche

1. Verfahren zur Herstellung von hochreinen weitgehend Fremdsalz-freien Ethersulfonaten der allgemeinen Formel I

$$R^1\text{-}[O\text{-}(CR^2R^{2'}\text{-}CR^3R^{3'})_x]_y\text{-}OCR^4R^{4'}CR^5R^5SO_3M \quad I$$

worin

| | |
|---|---|
| $R^1$ | Wasserstoff, $(C_1\text{-}C_{22})$-Alkyl, $(C_2\text{-}C_{22})$-Alkenyl, $(C_3\text{-}C_8)$-Cycloalkyl, $(C_6\text{-}C_{14})$-Aryl oder $(C_6\text{-}C_{14})$-Aryl-$(C_1\text{-}C_8)$-Alkyl bedeutet; |
| $R^2$ bis $R^5$ | sowie $R^{2'}$ bis $R^{5'}$ unabhängig voneinander Wasserstoff oder $(C_1\text{-}C_4)$-Alkyl bedeuten; |
| M | ein Alkalimetall, die Ammoniumgruppe oder eine substituierte Ammoniumgruppe bedeutet; |
| x | eine ganze Zahl von 1 bis 20 bedeutet; und |
| y | eine ganze Zahl von 0 bis 20 bedeutet, |

wobei für den Fall x>1 auch die Reste $R^2$, die Reste $R^{2'}$, die Reste $R^3$ und die Reste $R^{3'}$ jeweils unabhängig voneinander sind und wobei $R^1$ nicht Wasserstoff sein kann, wenn y = O durch Umsetzung einer Verbindung der allgemeinen Formel II

$$R^1\text{-}[O\text{-}(CR^2R^{2'}CR^3R^{3'})_x]_y\text{-}OH \quad II$$

mit einer Verbindung der allgemeinen Formel III

$$HO\text{-}CR^4R^{4'}CR^5R^{5'}\text{-}SO_3M \quad III$$

in Gegenwart einer Verbindung der allgemeinen Formel IV

$$MOH \quad IV$$

und anschließender Neutralisation der Verbindung der allgemeinen Formel IV mit einer Säure, dadurch gekennzeichnet, daß als Säure eine Verbindung der allgemeinen Formel V

$$R^1\text{-}[O\text{-}(CR^2R^{2'}\text{-}CR^3R^{3'})_x]_y\text{-}OCR^4R^{4'}CR^5R^{5'}SO_3H \quad V$$

eingesetzt wird.

2. Verfahren gemäß Anspruch 1, dadurch gekennzeichnet, daß

| | |
|---|---|
| $R^1$ | Wasserstoff oder $(C_1-C_6)$-Alkyl, |
| $R^2$ bis $R^5$ | bzw. $R^{2'}$ bis $R^{5'}$ unabhängig voneinander Wasserstoff oder $(C_1-C_3)$-Alkyl, |
| M | Natrium oder Kalium |
| x | eine ganze Zahl von 1 bis 4 und |
| y | eine ganze Zahl von 1 bis 10 |

bedeuten.

3.  Verfahren gemäß Anspruch 1 und/oder 2, dadurch gekennzeichnet, daß

| | |
|---|---|
| $R^1$ | Wasserstoff |
| $R^2$, $R^{2'}$, $R^3$ und $R^{3'}$ | unabhängig voneinander Wasserstoff oder Methyl, |
| $R^4$, $R^{4'}$, $R^5$ und $R^{5'}$ | Wasserstoff, |
| M | Natrium oder Kalium und |
| x und y | die Zahl 1 |

bedeuten.

4.  Verfahren gemäß einem oder mehreren der Ansprüche 1 bis 3, dadurch gekennzeichnet, daß die Verbindung der allgemeinen Formel II in einer Menge von 200 bis 700 Mol%, die Verbindung der allgemeinen Formel III in einer Menge von 100 Mol% und die Verbindung der allgemeinen Formel IV in einer Menge von 10 bis 20 Mol% eingesetzt werden.

5.  Verfahren gemäß einem oder mehreren der Ansprüche 1 bis 4, dadurch gekennzeichnet, daß nach Neutralisation des Reaktionsgemisches mit einer Verbindung der allgemeinen Formel V die im Überschuß eingesetzte Verbindung der allgemeinen Formel II mittels eines Dünnschichtverdampfers abgetrennt wird.